# EUROPEAN PATENT SPECIFICATION

(11) **EP 4 482 534 B1**
(45) Date of publication and mention of the grant of the patent: **23.09.2026**
(21) Application number: 23705004.2
(22) Date of filing: 16.02.2023
(51) Int. Cl.: A61L 2/00, A01K 1/00, A61L 2/10, A61L 9/20, A61N 5/06

(54) **UV-B LIGHTING SYSTEM AND METHOD**
UV-B-BELEUCHTUNGSSYSTEM UND -VERFAHREN
SYSTÈME ET PROCÉDÉS D'ÉCLAIRAGE UV-B

(30) Priority: 24.02.2022 EP 22158593
(43) Date of publication of application: 01.01.2025
(73) Proprietor: Signify Holding B.V., 5656 AE Eindhoven (NL)
(72) Inventor: VAN BOMMEL, Ties, 5656 AE Eindhoven (NL)
(74) Representative: Verweij, Petronella Daniëlle
(86) International application number: PCT/EP2023/053884
(87) International publication number: WO 2023/161119

(56) References cited:
- US-A1- 2018 015 192

## Description

### FIELD OF THE INVENTION

This invention relates to UV-B lighting systems and methods.

### BACKGROUND OF THE INVENTION

Various properties of UV lighting, and in particular UV-B lighting, have been widely researched. For the purposes of this disclosure, the UV-B wavelength range is defined to be 280nm to 320nm. This invention is of particular interest to lighting systems for delivering UV light, in particular UV-B light, to animals (e.g. humans or livestock).

It is well known that UV light stimulates the generation of Vitamin D in many animals, including humans as well as various livestock animals. For example, recent research has shown that the Vitamin D content in eggs can be increased by exposing chickens to UV-B light. UV-B exposure applied to laying hens during the later phase of the laying cycle has also been shown to promote health and welfare of laying hens. The UV-B radiation improves bone density, egg production, eggshell quality and yolk concentration.

Sterilizing properties of UV light are also well known. It is well known that UV light can be used for disinfection i.e. inactivating/killing of viruses and/or bacteria. US 2018/015192 A1 discloses a UV-B lighting system for disinfection and stimulation of vitamin D production.

Finally, while UV light is invisible to humans, different animals have different sensitivity to the wavelengths of the electromagnetic spectrum. This needs to be taken into account when devising a lighting program for example for livestock farming.

There is therefore a need for a UV-B lighting system with improved performance, functionality and/or safety.

### SUMMARY OF THE INVENTION

The invention is defined by the claims.

According to examples there is provided a (UV-B) lighting system comprising:
a first UV-B (solid-state) light source emitting, in operation, first UV-B light with a first dominant peak wavelength below 300nm;
a second UV-B (solid-state) light source emitting, in operation, second UV-B light with a second dominant peak wavelength above 300nm, wherein the dominant peak wavelengths differ by at least 10nm; and
a controller for controlling the output intensities delivered by the first and second UV-B (solid-state) light sources, wherein the controller is operable in:
   a first mode to generate a first light output with a first ratio, R1, of a first UV-B (solid-state) light source output intensity to a second UV-B (solid-state) light source output intensity; and
   a second mode to generate a second light output with a second ratio, R2, of the first UV-B (solid-state) light source output intensity to the second UV-B (solid-state) light source output intensity, wherein R1>R2.

Preferably, each of the output intensities delivered by the first and second UV-B (solid-state) light sources is individually controllable, and the controller is provided for individually controlling each of the output intensities of these light sources.

The UV-B lighting system according to the invention provides an improved performance, functionality and/or safety.

It is noted that the term "light" is used to refer generally to the electromagnetic spectrum, and specifically is intended to include the infrared, visible and ultraviolet part of the spectrum. Thus, a "light source" is not intended to be limited to a visible light source.

This lighting system enables control of the amounts of UV-B light in two wavelength bands/ranges. The UV-B light (across the full 280nm to 320nm width of the UV-B spectrum) is effective in promoting the generation of Vitamin D in various animals (including humans). However, it appears that the longer wavelength end of the UV-B wavelength range (e.g. 300nm to 320nm) is particularly suitable for destroying bacteria and is rather safe, but less suitable for inactivating viruses. It also appears that the shorter wavelength end of UV-B wavelength range (e.g. 280nm to 300nm) is, additional to killing bacteria, also particularly suitable for inactivating viruses, but is less safe (at high dose i.e. high intensity and/or exposure times). The dose (i.e. the light intensity times the exposure time) that can be administered safely depends on the wavelength, with longer wavelengths being less harmful than shorter wavelengths. Thus, the wavelength band with a dominant peak wavelength below 300nm is also particularly effective in destroying bacteria and viruses, but a high dose may not be desired as it is more harmful in creating tissue damage than longer wavelengths. The wavelength band with a dominant peak wavelength above 300nm is for example effective in destroying bacteria and can be safely applied with a higher intensity.

Furthermore, some livestock (such as birds or chickens) are able to perceive light in the longer UV wavelengths. The shorter wavelength end of the UV-B spectrum, hence the wavelength light output of the first UV-B light source, however remains invisible/unperceived and hence can be provided during the night (e.g. for stimulating Vitamin D production) whereas the first UV-B light source output may be providing, additionally or alternatively, during the day to better simulate the broad light spectrum of natural daylight. Thus, even within the UV-B band, some wavelengths may be visible to or sensed by some animals while others are not.

Furthermore, the allowable dose which can be applied in a safe way depends on the distance of the animal to the light source.

Furthermore, UV-B light sources emitting low wavelength UV-B light appear to be less efficient and/or more expensive.

The lighting system thus enables the performance, functionality and/or safety of the lighting system to be optimized for a particular purpose. The dominant peak wavelengths may differ by at least 15nm, for example by at least 20nm or even by at least 25nm.

The first ratio is greater than 1 and the second ratio is for example less than 1. Thus, a different one of the two light source outputs is dominant in each of the two modes.

In an example not part of the claimed invention, the first light output may have no second UV-B light source output and/or the second light output may have no first UV-B light source output. In other words, all of the light output in the first mode is the first light source output (below 300nm), hence the ratio is infinite, and/or all of the light in the second mode is the second light source output (above 300nm), hence the ratio is zero. This results in very different lighting quality or effect between the two modes, for example in terms of disinfection performance vs. safety or visibility (e.g. for birds such as poultry).

Instead, according to an example of the claimed invention, the first and second light outputs each have components from both the first and second UV-B light sources, in different proportions. Thus, the two modes may have different mixes of the two wavelengths. Thus, a balance can be optimized in terms of performance and safety.

A total intensity from the first and second solid state UV-B light sources (i.e. I1 + I2) is for example lower in the first mode than in the second mode. It is more safe to provide a higher intensity for the longer wavelength UV-B light of the second mode.

The first dominant peak wavelength is for example below 295 nm especially below 290nm and/or the second dominant peak wavelength is for example above 305 nm especially above 310nm. This provides spacing from the threshold wavelength of 300nm.

The lighting system may further comprise a white light source, wherein the controller is further for controlling the output intensity delivered by the white light source. The output intensity delivered by the white light source may be individually controllable, independent from the output intensities of the first and second UV-B (solid-state) light sources, and the controller may be provided for individually controlling the output intensity of the white light source. The white light source is used to better mimic the light spectrum during the day, for example with a combination of visible and UV-B light. The white light emitted by the white light source may have a correlated color temperature in a range from 2000K to 10000K and/or a color rendering index of at least 70 especially at least 80.

The first light output for example has no white light source output and the second light output has a white light source output component. The first light output is for example for use during darkness and the second light output is for example for use during the day.

The lighting system for example comprises a timer (i.e., a clock module) for operating the controller in the first mode and the second mode according to a time sequence. A user interface may be provided, wherein the timer is programmable via the user interface.

In one example, the time sequence provides the first light output during the night and the second light output during the day. For example, the first light output may be provided at least at one point during the night such as at or around midnight, and the second light output may be provided at least at one point during the day, such as at or around midday. Thus, natural lighting conditions as well as the intended purpose of the UV-B lighting, such as Vitamin D production and/or sterilization, are taken into account when scheduling and/or applying the time sequence.

In embodiments, said UV-B lighting system may comprise a light exit window for exiting the first UV-B light and the second UV-B light, and optionally the white light.

The lighting system for example comprises a livestock farming lighting system for example a bird or poultry farming lighting system.

In embodiments, the lighting system further comprises a sensor configured to detect at least one of a presence and a location of one or more animals and to determine a distance between (i) the one or more animals and (ii) a lighting arrangement comprising the first and second UV-B solid-state light sources, wherein the sensor is functionally coupled to the controller and the controller is further configured to control and adjust a ratio R between the first UV-B solid state light source output intensity and the second UV-B solid state light source output intensity as a function of the determined distance between the one or more animals and the lighting arrangement.

In embodiments, the controller is configured to decrease the ratio when the determined distance is below a predefined threshold (e.g. <3m) and to increase the ratio when the determined distance is above the predefined threshold (e.g. >3m).

In embodiments, the controller is arranged to decrease the ratio by increasing the intensity of the second UV-B solid-state light source output intensity, and to increase the ratio by decreasing the intensity of the second UV-B solid-state light source output intensity.

In embodiments, the controller is arranged to decrease the ratio by decreasing the intensity of the first UV-B solid-state light source output intensity, and to increase the ratio by increasing the intensity of the first UV-B solid-state light source output intensity.

In embodiments, the controller is arranged to decrease the ratio by increasing the intensity of the second UV-B solid-state light source output intensity and decreasing the intensity of the first UV-B solid-state light source output intensity, and to increase the ratio by decreasing the intensity of the second UV-B solid-state light source output intensity and by increasing the intensity of the first UV-B solid-state light source output intensity.

In embodiments, when the ratio is decreased the total intensity (i.e. the first and second UV-B solid-state light source output intensity) is increased and when the ratio is increased the total intensity (i.e. the first and second UV-B solid-state light source output intensity) is decreased.

In embodiments, the UV-B solid-state light source output intensity is zero if the one or more animals are within the predefined threshold (e.g. <3m) for the determined distance between the one or more animals and the lighting arrangement (i.e. the first and second UV-B solid-state light source).

The invention also provides a method of controlling a UV-B lighting system, the lighting system comprising a first UV-B solid state light source emitting, in operation, a first dominant peak wavelength below 300nm and a second solid state UV-B light source emitting, in operation, a second dominant peak wavelength above 300nm, wherein the dominant peak wavelengths differ by at least 10nm, wherein the method comprises:
controlling the output intensities delivered by the first and second solid state UV-B light sources such that:
at a first time a first light output is generated with a first ratio, R1, of the first UV-B solid state light source output intensity to the second UV-B solid state light source output intensity; and
at a second time a second light output is generated with a second ratio, R2, of the first UV-B solid state light source output intensity to the second UV-B solid state light source output intensity, wherein wherein R1>R2.

According to the claimed method the first and second light outputs each have components from both the first and second UV-B light sources, in different proportions. Thus, the two modes may have different mixes of the two wavelengths. Thus, a balance can be optimized in terms of performance and safety.

The first time is for example during the night (or other resting time of livestock) and then the first light source output is dominant. The second time is for example during the day and then the second light source output is dominant.

The method may comprise providing the first and second light outputs to livestock of a livestock farming system, such as to poultry of a poultry farming system.

The disclosure also provides a computer program comprising computer program code means which is adapted, when said program is run on a computer, to implement the method defined above.

The disclosed (UV-B) lighting system and method of controlling such (UV-B) lighting system are especially suitable for use in agriculture applications such as livestock farming (e.g., poultry farming) and the (UV-B) lighting system may be configured to provide illumination to livestock (e.g., poultry) in a farm. The disclosed (UV-B) lighting system may therefore also be referred to as an agriculture (UV-B) lighting system such as a livestock farming (UV-B) lighting system (e.g., a poultry farming (UV-B lighting system)). For example, the disclosed (UV-B) lighting system may be configured to comply with certain ingress protection rating (IP Rating) requirements for agriculture environment such as livestock farming environments (e.g., poultry farming environments). Some disclosed (UV-B) lighting systems may be configured for high bay application in agriculture such as livestock farming (e.g., poultry farming).

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a lighting system;
Fig. 2 shows sensitivity to light wavelengths; and
Fig. 3 shows a lighting control method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a UV-B lighting system which has first and second UV-B light sources with different dominant peak wavelengths (one above 300nm and one below 300nm). In a first mode, light is generated with a first ratio of the two light source output intensities and in a second mode, light is generated with a second, different, ratio of the light source output intensities. The goals of sterilization, Vitamin D production, safety, and simulation of natural lighting conditions can thereby be balanced.

The invention makes use of solid state light sources. These will however simply each be referred to as a "light source" in the description below.

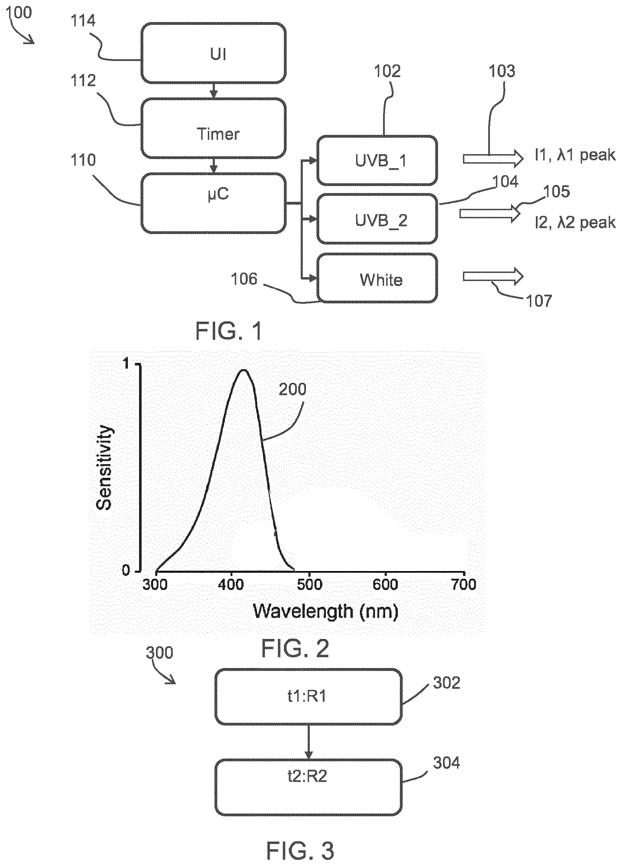

Figure 1 shows UV-B lighting system 100 comprising a first UV-B light source 102 with a first dominant peak wavelength λ1 (i.e. wavelength at peak intensity) below 300nm and a second UV-B light source 104 with a second dominant peak wavelength λ2 above 300nm. The first UV-B light source 102 delivers a light output 103 with an output intensity I1 and the second UV-B light source 104 delivers a light output 105 with an output intensity I2. These intensities are controllable at least between on and off, and optionally also between analogue intensity levels. Preferably, each of these intensities is individually controllable. The total UV-B intensity is thus I1+I2.

In a preferred example, the first dominant peak wavelength is below 290nm and the second dominant peak wavelength is above 310nm.

The dominant peak wavelength is the wavelength at which the light output intensity is highest. In practice, each light source will generate a band of wavelengths, with the dominant peak wavelength near the center of the band. These bands may overlap. However, the dominant peak wavelengths differ by at least 10nm, preferably at least 15 nm, more preferably at least 20 nm, most preferably at least 25 nm. A larger difference may be better in terms of disinfection performance vs. safety or visibility (e.g. for poultry).

A controller 110 is provided for controlling the output intensities delivered by the first and second UV-B light sources 102, 104. Preferably, the controller is provided for individually controlling each of the output intensities of the light sources. In particular, a different combination of the two wavelength bands may be provided at different times, to achieve different lighting aims.

There may be multiple combinations of different light intensities from the two light sources 102, 104, giving multiple operating modes. However, as a minimum, there are at least two modes.

In the first mode, a first light output is generated with a first ratio R1 of the first UV-B light source output intensity to the second UV-B light source output intensity. This ratio R1 is for example greater than 1, meaning that the output intensity of the first light source (below 300nm) is dominant.

In the second mode, a second light output is generated with a second, different, ratio R2 of the first UV-B light source output intensity to the second UV-B light source output intensity, This ratio R2 is for example less than 1, meaning that the output intensity of the second light source (above 300nm) is dominant.

In one example, not part of the claimed invention, only one of the two light sources is turned on at any time. Thus, the first light output has no second UV-B light source output (R1 = ∞) and the second light output has no first UV-B light source output (R2 = 0).

Instead, according to the invention, both light sources are on for one or both of the modes so that 1 < R1 < ∞ for the first light output and 0 < R2 < 1 for the second light output.

A total intensity from the first and second solid state UV-B light sources (i.e. I1 + I2) may be controlled to be lower in the first mode than in the second mode. It is more safe to provide a higher intensity for the longer wavelength UV-B light of the second mode.

Figure 1 also shows a white light source 106 controlled by the controller for delivering a white light output 107.

The system thus enables control of the amounts of UV-B light in two wavelength bands to be controlled. The particular characteristics of the light output at any time are chosen to achieve one or more of several objectives:
Vitamin D production;
Bacteria sterilization;
Virus sterilization;
Mimicking natural daylight (as perceived by a target animal species);
Mimicking natural night light (as perceived by a particular target animal species, e.g. avoiding disturbance during animal rest);
Ensuring a safe UV dose.

By way of example, it has been shown that chickens can perceive light down to wavelengths of around 300nm. Thus, to avoid disturbance during the night or rest times, the UV-B light above 300nm is not desired. However, during the day, this part of the spectrum is present in sunlight so is desirable to better mimic the broad spectrum of natural sunlight.

The first light output (with dominant peak wavelength below 300nm) for example has no white light source output component since this may be used during darkness, whereas the second light output (with dominant peak wavelength above 300nm) has a white light source output component.

Thus, the first light output has no white light and R1>1 whereas the second light output has white light and R2<1.

Thus, it can be seen that the two or more modes may be applied as a function of the time of day. For this purpose, Figure 1 shows a timer 112 (i.e., clock) for operating the controller in the first mode and the second mode in accordance with a time sequence. A user interface 114 is for example used to enable a user of the system to program the timer. Thus user interface may be implemented by an app loaded onto a mobile phone. The program determines a value of R, such as R1 and R2 referred to above, for different time slots as well as the white light intensity (when the optional white light source is used).

The white light may have an intensity and/or color properties (e.g., color temperature) that varies over time, again to mimic natural lighting conditions such as dawn and sunset. The white light source may be individually controllable independent from the first UV-B light source and the second UV-B light source.

Figure 2 shows a plot of the sensitivity to light in the wavelength range 300nm to 700nm for chickens. It shows that the sensitivity starts at 300nm.

The control of the UV-B light may also take account of the distance between the lighting arrangement (comprising the first and second UV-B solid-state light sources) and animals in an enclosure. In this way, the dose of the different light delivered to the animals may be controlled, also taking account of the different attenuation for different wavelengths. For this purpose, a sensor may be provided to detect at least one of a presence and a location of one or more animals and to determine a distance between (i) the one or more animals and (ii) the lighting arrangement.

The controller may then control and adjust a ratio between the first UV-B solid state light source output intensity and the second UV-B solid state light source output intensity as a function of the determined distance between the one or more animals and the lighting arrangement.

The ratio is for example decreased (so relatively less of the first UV-B light) when the animals are close, i.e., the determined distance is below a predefined threshold, and increased (so relatively more of the first UV-B light) when the determined distance is above the predefined threshold.

The threshold is for example 3m. The ratio may be decreased by increasing the intensity of the second UV-B solid-state light source output intensity, and the ratio may be increased by decreasing the intensity of the second UV-B solid-state light source output intensity.

Alternatively, the ratio may be decreased by decreasing the intensity of the first UV-B solid-state light source output intensity, and the ratio may be increased by increasing the intensity of the first UV-B solid-state light source output intensity.

Alternatively, the ratio may be decreased by increasing the intensity of the second UV-B solid-state light source output intensity and decreasing the intensity of the first UV-B solid-state light source output intensity, and the ratio may be increased by decreasing the intensity of the second UV-B solid-state light source output intensity and by increasing the intensity of the first UV-B solid-state light source output intensity.

When the ratio is decreased, the total intensity may be increased and when the ratio is increased the total intensity may be decreased.

The UV-B solid-state light source output intensity may be made zero if the one or more animals are within the (or another) predefined distance threshold.

Figure 3 shows a method 300 of controlling the UV-B lighting system as described above. The method comprises controlling the output intensities delivered by the first and second UV-B light sources such that:
in step 302, at a first time, a first light output is generated with a first ratio R1 of the first UV-B light source output intensity to the second UV-B light source output intensity; and
in step 304, at a second time, a second light output is generated with a second, different, ratio R2 of the first UV-B light source output intensity to the second UV-B light source output intensity.

The control of the light sources is for example implemented by software running on the controller.

The switching between modes may take place between day and night or it may take place with greater frequency, for example such that the daytime is divided into different time zones (e.g. resting and active times). Indeed, a program of values of R for a ratio of the first UV-B light source output intensity to the second UV-B light source output intensity may be divided into time slots of any desired resolution (minutes or hours). For example, the time periods during which the ratio is set at a given value may last at least 1 minute, more preferably at least 10 minutes, and most preferably at least 1 hour. The time periods are for example less than 22 hours, more preferably less than 20 hours, most preferably less than 18 hours. There may for example be a gradual change in the ratio over time.

The light sources for example comprise UV-LEDs and/or UV laser diodes.

The lighting system may include additional light sources to those shown, such as UV-A light sources. However, the first light output and/or the second light output may for example not comprise UV-A light (in the range 320nm to 400nm) and/or UV-C light (in the range 100nm to 280 nm).

Furthermore, the lighting system may be combined with a ventilation and/or heating system to provide an overall system for controlling the environmental conditions, for example of farming livestock.

Sensors may be provided for automating or partially automating the control of the lighting system. The lighting modes may be selected based on sensed conditions (e.g. activity levels) of the livestock.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A agricultural UV-B lighting system (100) comprising:
a first UV-B solid-state light source (102) emitting, in operation, first UV-B light (103) with a first dominant peak wavelength (λ1) below 300nm;
a second UV-B solid-state light source (104) emitting, in operation, second UV-B light (105) with a second dominant peak wavelength (λ2) above 300nm, wherein the dominant peak wavelengths differ by at least 10nm; and
a controller (110) for individually controlling the output intensities delivered by the first and second UV-B solid-state light sources (102,104), wherein the controller is operable in:
a first mode to generate a first light output with a first ratio, R1, of a first UV-B solid-state light source output intensity (I1) to a second UV-B solid-state light source output intensity (I2); and
a second mode to generate a second light output with a second ratio, R2, of the first UV-B solid-state light source output intensity (I1) to the second UV-B solid-state light source output intensity (I2), wherein R1>R2,
wherein the first and second light outputs each have components from both the first and second UV-B light sources.

2. The agricultural lighting system of claim 1, wherein the first ratio, R1, is greater than 1 and the second ratio, R2, is less than 1.

3. The agricultural lighting system of any one of claims 1 to 2 wherein a total intensity from the first and second solid state UV-B light sources is lower in the first mode than in the second mode.

4. The agricultural lighting system of any one of claims 1 to 3, wherein the first dominant peak wavelength is below 290nm and/or the second dominant peak wavelength is above 310nm.

5. The agricultural lighting system of any one of claims 1 to 4 further comprising a white solid-state light source (106) emitting, in operation, white light (107), wherein the controller is further configured for controlling the output intensity delivered by the white solid-state light source.

6. The agricultural lighting system of claim 5, wherein the first light output has no white light and the second light output has white light.

7. The agricultural lighting system of any one of claims 1 to 6, comprising a timer (112) configured to operate the controller in the first mode and the second mode according to a time sequence; and a user interface (114), wherein the timer (112) is configured to be programmable with the time sequence via the user interface.

8. The agricultural lighting system of claim 7 wherein the time sequence provides the first light output during the night and the second light output during the day.

9. The agricultural lighting system according to any one of the preceding claims, further comprising a sensor configured to detect at least one of a presence and a location of one or more animals and to determine a distance between (i) the one or more animals and (ii) a lighting arrangement comprising the first and second UV-B solid-state light sources, wherein the controller is further configured to control and adjust a ratio R between the first UV-B solid state light source output intensity and the second UV-B solid state light source output intensity as a function of the determined distance between the one or more animals and the lighting arrangement.

10. The agricultural lighting system of claim 9, wherein the controller is configured to decrease a ratio R between the first UV-B solid state light source output intensity and the second UV-B solid state light source output intensity when the determined distance is below a predefined threshold and to increase the ratio R when the determined distance is above the predefined threshold

11. The agricultural lighting system of any one of claims 1 to 10 wherein the agricultural lighting system is a livestock farming lighting system such as a poultry farming lighting system.

12. A method (300) of controlling an agricultural UV-B lighting system, the lighting system comprising a first UV-B solid state light source emitting, in operation, a first dominant peak wavelength below 300nm and a second solid state UV-B light source emitting, in operation, a second dominant peak wavelength above 300nm, wherein the dominant peak wavelengths differ by at least 10nm, wherein the method comprises:
individually controlling the output intensities delivered by the first and second solid state UV-B light sources such that:
(302) at a first time a first light output is generated with a first ratio, R1, of the first UV-B solid state light source output intensity to the second UV-B solid state light source output intensity; and
(304) at a second time a second light output is generated with a second ratio, R2, of the first UV-B solid state light source output intensity to the second UV-B solid state light source output intensity, wherein R1>R2
wherein the first and second light outputs each have components from both the first and second UV-B light sources.

13. The method (300) of claim 12 wherein the agricultural UV-B lighting system is a livestock lighting system, the method further comprising controlling the output intensities delivered by the first and second solid state UV-B light sources such that the first time occurs during a night or resting time of a livestock and the second time occurs during the day of the livestock.

## Patentansprüche

1. Landwirtschaftliches UV-B-Beleuchtungssystem (100), umfassend:
eine erste UV-B-Festkörperlichtquelle (102), die, in Betrieb, erstes UV-B-Licht (103) mit einer ersten farbtongleichen Spitzenwellenlänge (λ1) unter 300 nm emittiert;
eine zweite UV-B-Festkörper-Lichtquelle (104), die, in Betrieb, zweites UV-B-Licht (105) mit einer zweiten farbtongleichen Spitzenwellenlänge (λ2) über 300 nm emittiert, wobei sich die farbtongleichen Spitzenwellenlängen um mindestens 10 nm unterscheiden; und
eine Steuerung (110) zum individuellen Steuern der Ausgabeintensitäten, die durch die erste und die zweite UV-B-Festkörperlichtquelle (102, 104) abgegeben werden, wobei die Steuerung betreibbar ist in:
einem ersten Modus, um eine erste Lichtausgabe mit einem ersten Verhältnis, R1, von einer Ausgabeintensität (11) einer ersten UV-B-Festkörperlichtquelle zu einer Ausgabeintensität (12) einer zweiten UV-B-Festkörperlichtquelle zu erzeugen; und
einem zweiten Modus, um eine zweite Lichtausgabe mit einem zweiten Verhältnis, R2, von der Ausgabeintensität (11) der ersten UV-B-Festkörperlichtquelle zu der Ausgabeintensität (12) der zweiten UV-B-Festkörperlichtquelle zu erzeugen, wobei R1>R2,
wobei die erste und die zweite Lichtausgabe jeweils Komponenten sowohl von der ersten als auch von der zweiten UV-B-Lichtquelle aufweisen.

2. Landwirtschaftliches Beleuchtungssystem nach Anspruch 1, wobei das erste Verhältnis, R1, größer als 1 ist und das zweite Verhältnis, R2, kleiner als 1 ist.

3. Landwirtschaftliches Beleuchtungssystem nach einem der Ansprüche 1 bis 2, wobei eine Gesamtintensität von der ersten und der zweiten Festkörper-UV-B-Lichtquelle in dem ersten Modus geringer als in dem zweiten Modus ist.

4. Landwirtschaftliches Beleuchtungssystem nach einem der Ansprüche 1 bis 3, wobei die erste farbtongleiche Spitzenwellenlänge unter 290 nm liegt und/oder die zweite farbtongleiche Spitzenwellenlänge über 310 nm liegt.

5. Landwirtschaftliches Beleuchtungssystem nach einem der Ansprüche 1 bis 4, ferner umfassend eine weiße Festkörperlichtquelle (106), die, in Betrieb, weißes Licht (107) emittiert, wobei die Steuerung ferner zum Steuern der Ausgabeintensität konfiguriert ist, die durch die weiße Festkörperlichtquelle abgegeben wird.

6. Landwirtschaftliches Beleuchtungssystem nach Anspruch 5, wobei die erste Lichtausgabe kein weißes Licht aufweist und die zweite Lichtausgabe weißes Licht aufweist.

7. Landwirtschaftliches Beleuchtungssystem nach einem der Ansprüche 1 bis 6, umfassend einen Zeitgeber (112), der konfiguriert ist, um die Steuerung gemäß einer zeitlichen Abfolge in dem ersten Modus und dem zweiten Modus zu betreiben; und eine Bedienerschnittstelle (114), wobei der Zeitgeber (112) konfiguriert ist, um über die Bedienerschnittstelle mit der Zeitsequenz programmierbar zu sein.

8. Landwirtschaftliches Beleuchtungssystem nach Anspruch 7, wobei die Zeitsequenz die erste Lichtausgabe während der Nacht und die zweite Lichtausgabe während des Tages bereitstellt.

9. Landwirtschaftliches Beleuchtungssystem nach einem der vorstehenden Ansprüche, ferner umfassend einen Sensor, der konfiguriert ist, um mindestens eines von einer Anwesenheit und einer Position von einem oder mehreren Tieren zu erfassen und einen Abstand zwischen (i) dem einen oder den mehreren Tieren und (ii) einer Beleuchtungsanordnung zu bestimmen, umfassend die erste und die zweite UV-B-Festkörperlichtquelle, wobei die Steuerung ferner konfiguriert ist, um ein Verhältnis R zwischen der Ausgabeintensität der ersten UV-B-Festkörperlichtquelle und der Ausgabeintensität der zweiten UV-B-Festkörperlichtquelle in Abhängigkeit des bestimmten Abstands zwischen dem einen oder den mehreren Tieren und der Beleuchtungsanordnung zu steuern und anzupassen.

10. Landwirtschaftliches Beleuchtungssystem nach Anspruch 9, wobei die Steuerung konfiguriert ist, um ein Verhältnis R zwischen der Ausgabeintensität der ersten UV-B-Festkörperlichtquelle und der Ausgabeintensität der zweiten UV-B-Festkörperlichtquelle zu verringern, wenn der bestimmte Abstand unter einem vordefinierten Schwellenwert liegt, und das Verhältnis R zu erhöhen, wenn der bestimmte Abstand über dem vordefinierten Schwellenwert liegt

11. Landwirtschaftliches Beleuchtungssystem nach einem der Ansprüche 1 bis 10, wobei das landwirtschaftliche Beleuchtungssystem ein Beleuchtungssystem für die Nutztierhaltung ist, wie ein Beleuchtungssystem für die Geflügelhaltung.

12. Verfahren (300) zum Steuern eines landwirtschaftlichen UV-B-Beleuchtungssystems, das Beleuchtungssystem umfassend eine erste UV-B-Festkörperlichtquelle, die, in Betrieb, eine erste farbtongleiche Spitzenwellenlänge unter 300 nm emittiert, und eine zweite Festkörper-UV-B-Lichtquelle, die, in Betrieb, eine zweite farbtongleiche Spitzenwellenlänge über 300 nm emittiert, wobei sich die farbtongleichen Spitzenwellenlängen um mindestens 10 nm unterscheiden, wobei das Verfahren umfasst:
derartiges individuelles Steuern der Ausgabeintensitäten, die durch die erste und die zweite Festkörper-UV-B-Lichtquelle abgegeben werden, dass:
(302) zu einem ersten Zeitpunkt eine erste Lichtausgabe mit einem ersten Verhältnis, R1, der Ausgabeintensität der ersten UV-B-Festkörperlichtquelle zu der Ausgabeintensität der zweiten UV-B-Festkörperlichtquelle erzeugt wird; und
(304) zu einem zweiten Zeitpunkt eine zweite Lichtausgabe mit einem zweiten Verhältnis, R2, der Ausgabeintensität der ersten UV-B-Festkörperlichtquelle zu der Ausgabeintensität der zweiten UV-B-Festkörperlichtquelle erzeugt wird, wobei R1>R2
wobei die erste und die zweite Lichtausgabe jeweils Komponenten sowohl von der ersten als auch von der zweiten UV-B-Lichtquelle aufweisen.

13. Verfahren (300) nach Anspruch 12, wobei das landwirtschaftliche UV-B-Beleuchtungssystem ein Nutztierbeleuchtungssystem ist, das Verfahren ferner umfassend das derartige Steuern der Ausgabeintensitäten, die durch die erste und die zweite Festkörper-UV-B-Lichtquellen abgegeben werden, dass der erste Zeitpunkt während einer Nacht- oder Ruhezeit eines Nutztieres auftritt und der zweite Zeitpunkt während des Tages des Nutztieres auftritt.

## Revendications

1. Système d'éclairage UV-B agricole (100) comprenant :
une première source de lumière à semi-conducteurs UV-B (102) émettant, en fonctionnement, de la première lumière UV-B (103) avec une première longueur d'onde de pic dominante (λ1) inférieure à 300 nm ;
une seconde source de lumière à semi-conducteurs UV-B (104) émettant, en fonctionnement, de la seconde lumière UV-B (105) avec une seconde longueur d'onde de pic dominante (λ2) supérieure à 300 nm, dans lequel les longueurs d'onde de pic dominantes diffèrent d'au moins 10 nm ; et
un organe de commande (110) permettant de commander individuellement les intensités de sortie délivrées par les première et seconde sources de lumière à semi-conducteurs UV-B (102, 104), dans lequel l'organe de commande est fonctionnel dans :
un premier mode pour générer une première sortie de lumière avec un premier rapport, R1, d'une intensité de sortie de première source de lumière à semi-conducteurs UV-B (11) à une intensité de sortie de seconde source de lumière à semi-conducteurs UV-B (12) ; et
un second mode pour générer une seconde sortie de lumière avec un second rapport, R2, de l'intensité de sortie de première source de lumière à semi-conducteurs UV-B (11) à l'intensité de sortie de seconde source de lumière à semi-conducteurs UV-B (12), dans lequel R1>R2,
dans lequel les première et seconde sorties de lumière ont chacune des composantes provenant à la fois des première et seconde sources de lumière UV-B.

2. Système d'éclairage agricole selon la revendication 1, dans lequel le premier rapport, R1, est supérieur à 1 et le second rapport, R2, est inférieur à 1.

3. Système d'éclairage agricole selon l'une quelconque des revendications 1 à 2 dans lequel une intensité totale provenant des première et seconde sources de lumière UV-B à semi-conducteurs est plus basse dans le premier mode que dans le second mode.

4. Système d'éclairage agricole selon l'une quelconque des revendications 1 à 3, dans lequel la première longueur d'onde de pic dominante est inférieure à 290 nm et/ou la seconde longueur d'onde de pic dominante est supérieure à 310 nm.

5. Système d'éclairage agricole selon l'une quelconque des revendications 1 à 4 comprenant en outre une source de lumière blanche à semi-conducteurs (106) émettant, en fonctionnement, de la lumière blanche (107), dans lequel l'organe de commande est configuré en outre pour commander l'intensité de sortie délivrée par la source de lumière blanche à semi-conducteurs.

6. Système d'éclairage agricole selon la revendication 5, dans lequel la première sortie de lumière n'a aucune lumière blanche et la seconde sortie de lumière a de la lumière blanche.

7. Système d'éclairage agricole selon l'une quelconque des revendications 1 à 6, comprenant un temporisateur (112) configuré pour faire fonctionner l'organe de commande dans le premier mode et le second mode selon une séquence temporelle ; et une interface utilisateur (114), dans lequel le temporisateur (112) est configuré pour être programmable avec la séquence temporelle par l'intermédiaire de l'interface utilisateur.

8. Système d'éclairage agricole selon la revendication 7 dans lequel la séquence temporelle fournit la première sortie de lumière pendant la nuit et la seconde sortie de lumière pendant la journée.

9. Système d'éclairage agricole selon l'une quelconque des revendications précédentes, comprenant en outre un capteur configuré pour détecter au moins l'une parmi une présence et une localisation d'un ou plusieurs animaux et pour déterminer une distance entre (i) le ou les animaux et (ii) un agencement d'éclairage comprenant les première et seconde sources de lumière à semi-conducteurs, dans lequel l'organe de commande est configuré en outre pour commander et ajuster un rapport R entre l'intensité de sortie de première source de lumière à semi-conducteurs UV-B et l'intensité de sortie de seconde source de lumière à semi-conducteurs UV-B en fonction de la distance déterminée entre le ou les animaux et l'agencement d'éclairage.

10. Système d'éclairage agricole selon la revendication 9, dans lequel l'organe de commande est configuré pour diminuer un rapport R entre l'intensité de sortie de première source de lumière à semi-conducteurs UV-B et l'intensité de sortie de seconde source de lumière à semi-conducteurs UV-B lorsque la distance déterminée est inférieure à un seuil prédéfini et pour augmenter le rapport R lorsque la distance déterminée est supérieure au seuil prédéfini

11. Système d'éclairage agricole selon l'une quelconque des revendications 1 à 10 dans lequel le système d'éclairage agricole est un système d'éclairage d'élevage d'animaux d'élevage tel qu'un système d'éclairage d'élevage de volailles.

12. Procédé (300) de commande d'un système d'éclairage UV-B agricole, le système d'éclairage comprenant une première source de lumière à semi-conducteurs UV-B émettant, en fonctionnement, une première longueur d'onde de pic dominante inférieure à 300 nm et une seconde source de lumière UV-B à semi-conducteurs émettant, en fonctionnement, une seconde longueur d'onde de pic dominante supérieure à 300 nm, dans lequel les longueurs d'onde de pic dominantes diffèrent d'au moins 10 nm, dans lequel le procédé comprend :
la commande individuelle des intensités de sortie délivrées par les première et seconde sources de lumière UV-B à semi-conducteurs de telle sorte que :
(302) à un premier moment une première sortie de lumière est générée avec un premier rapport, R1, de l'intensité de sortie de première source de lumière à semi-conducteurs UV-B à l'intensité de sortie de seconde source de lumière à semi-conducteurs UV-B ; et
(304) à un second moment une seconde sortie de lumière est générée avec un second rapport, R2, de l'intensité de sortie de première source de lumière à semi-conducteurs UV-B à l'intensité de sortie de seconde source de lumière à semi-conducteurs UV-B, dans lequel R1>R2
dans lequel les première et seconde sorties de lumière ont chacune des composantes provenant à la fois des première et seconde sources de lumière UV-B.

13. Procédé (300) selon la revendication 12 dans lequel le système d'éclairage UV-B agricole est un système d'éclairage d'animaux d'élevage, le procédé comprenant en outre la commande des intensités de sortie délivrées par les première et seconde sources de lumière UV-B à semi-conducteurs de telle sorte que le premier moment se produit pendant la nuit ou un moment de repos d'un animal d'élevage et le second moment se produit pendant la journée de l'animal d'élevage.
